# EUROPEAN PATENT SPECIFICATION

(11) **EP 4 301 297 B1**
(45) Date of publication and mention of the grant of the patent: **29.07.2026**
(21) Application number: 22765982.8
(22) Date of filing: 03.03.2022
(51) Int. Cl.: A61F 5/56

(54) **AN INTELLIGENT INTRA-ORAL DEVICE**
INTELLIGENTE INTRAORALE VORRICHTUNG
DISPOSITIF INTRA-ORAL INTELLIGENT

(30) Priority: 08.03.2021 AU 2021900648
(43) Date of publication of application: 10.01.2024
(73) Proprietor: SomnoMed Limited, Crows Nest, New South Wales 2065 (AU)
(72) Inventor: BEDFORD, Christopher Russell, Rozelle, New South Wales 2039 (AU); WOOD, Harrison John, Ourimbah, New South Wales 2258 (AU); MEIER, Joshua Luke, Berowra, New South Wales 2081 (AU); OBUCHI, Wataru, Maroubra, New South Wales 2035 (AU)
(74) Representative: Bennett, Adrian Robert J.
(86) International application number: PCT/AU2022/050171
(87) International publication number: WO 2022/187889

(56) References cited:
- WO-A1-2017/106840
- WO-A1-2018/098527
- WO-A2-2014/159236
- US-A1- 2009 078 273
- US-A1- 2014 152 464
- US-A1- 2016 199 215
- US-A1- 2017 143 259
- US-A1- 2017 196 727
- US-A1- 2020 197 655
- US-A1- 2020 375 528

## Description

### Technical Field

The present invention relates generally to an intra-oral device and, in particular, to detect the use of the intra-oral device and medical events of a patient when the intra-oral device is being used.

### Background

Globally, almost 1.5 billion people have Obstructive Sleep Apnea (OSA). OSA is a condition whereby the airway at the back of the throat collapses during sleep, reducing and potentially stopping, airflow. OSA has long term medical implications including increased risk of heart failure, hypertension, type 2 diabetes and stroke. It is estimated that 80% of patients are unaware they have this condition. Patients who show symptoms of OSA are monitored during their sleep with a polysomnography (PSG), which determines the existence and severity of OSA. PSG is typically completed via an overnight stay in hospital, so is expensive and often rationed by healthcare systems. This leads to patients with symptoms remaining undiagnosed for longer periods.

Around 85% of OSA patients are typically treated with Continuous Positive Airway Pressure (CPAP). CPAP therapy requires air to be pumped into the airway of a patient to inflate, and by doing so, keeping that airway open. To do so, the patient is required to wear a mask that is tightly sealed over the nose and mouth of the patient, creating a cavity between the mask and a portion of the patient's face. An air pump then pumps air, via a hose, into the cavity while the patient is asleep. The patient is forced to intake the air pumped into the cavity.

Apnea events are generally measured by an Apnea Hypopnea Index (AHI), which is the number of apneas or hypopneas recorded per hour of sleep.

CPAP typically reduces a patient's AHI to less than 5 events per hour (which is considered fully treated). However, wearing a CPAP mask can be uncomfortable, as patients can become entangled in the hose and the noise of the CPAP air pump can keep the patient and their bed partner awake. Therefore, compliance to CPAP therapy has been shown to be low. Most patients do not wear their CPAP device for the full night's sleep, or on every night during a typical week. To be considered to be compliant to therapy, a patient typically needs to use CPAP for at least 4 hours/night on 5 out of 7 nights per week. During the time the patient is sleeping but not using their CPAP device, the patient continues to have apneas and the elevated medical risk resulting from OSA equally continues.

Compliance of CPAP therapy can be tracked through measurements of airflow and pressure by the CPAP device. Efficacy of CPAP therapy can be measured by placing a sensor (e.g., for detecting breathing activity) on the mask. The measurements are sent back to the patients, physicians, and equipment suppliers daily via cloud-based software. Physicians can then modify treatment (e.g., change masks, add humidification, etc.) to improve compliance and efficacy.

The lack of a comfortable CPAP device and its low compliance have led to the development of a number of alternative therapies, including Oral Appliance Therapy (OAT). An example of OAT is a mouthguard type appliance that moves the lower jaw and tongue forward to open the airway, thereby reducing a patient's number of apnea events or their AHI. Appliances for OAT are designed to be comfortable while in the mouth and are typically wom for most of the night and all nights of the week. Therefore, compliance to OAT is considerably higher than with CPAP.

However, unlike a CPAP device, tracking compliance to and the efficacy of OAT, is more difficult. Accordingly, there is little capability for OAT appliances to provide feedback to the physician or patient on the efficacy and compliance of a patient's OAT in treating their OSA on an ongoing and longer term basis.

For example, an OAT device may use a pressure sensor or a temperature sensor to determine whether a patient is using the OAT device. This data however is often inaccurate as that patient is not asleep at least in the beginning and may be awake in the middle of the night due to an apnea event. Further, a sensor may be used to track biometric information of the patient to attempt to determine the AHI of a patient. However, due to the inaccuracy of the compliance data, the resulting efficacy data is also inaccurate.

Individual patient feedback is of critical importance because sleep apnea is heterogeneous in the population. Evidence shows that there is not one OAT design that most effectively improves polysomnographic indices, but that efficacy depends on a number of factors including severity of OSA, materials and method of fabrication, type of OAT device, and the degree of protrusion (sagittal and vertical).

Complicating sleep apnea treatments is the fact that an individual's sleep patterns can change from night to night. The position that a patient sleeps in, being on one's back, stomach or side can affect the efficacy of OAT treatment.

Furthermore, the success of OAT can be affected by numerous factors outside of an individual appliance design, for example weight gain/loss. These factors can change over time and therefore it becomes important to enable easy and objective monitoring of these physiological changes over time. With this information, a physician can coach the patient to undertake weight loss or modify their treatment and can involve the patient in the solution.

Sleep apnea has no cure and is a disease that generally worsens over time when left untreated. Therefore, there is a need for ongoing longer term monitoring of compliance to and efficacy of OAT.

By gaining individualised feedback, every patient can customise their oral appliance therapy to achieve the maximum possible effectiveness, minimise undesirable side effects and continuously optimise their individual treatment based upon objectively measured parameters.

For example, OAT is known to work largely, although not exclusively, in a dose-dependent manner. Thus, the further the patients lower jaw is advanced (moved in forward direction) the greater the likelihood of therapeutic success. Whilst this may be true for the majority of patients, there are other patients who require very little protrusion to maximise therapeutic success. The dose-dependent effect of mandibular protrusion on reduction of AHI by mandibular advancement devices is nonlinear and becomes more pronounced with increased severity of OSA. The mandibular protrusion should be more personalized to each patient. Therefore, the nightly monitoring of an individual patient's biological signals will allow the correct protrusion to be set for a patient, which will personalise treatment and ensure therapeutic success in OAT.

Complicating the ideal protrusive setting formula is the fact that one of the possible side effects of appliance therapy is undesirable tooth movement. It has been demonstrated that tooth movement occurs in a similar dose-dependent manner whereby the further forward the mandible is protruded the greater the forces translated to the teeth resulting in tooth movement. Therefore it would be advantageous to know how far forward the lower jaw is being protruded at the same time as knowing how effective the therapy is at the given degree of protrusion. A physician may subsequently balance the degree of successful therapy against the potential side effects if efficacy and compliance data is available on a night-to-night basis. A logical extension of this technology may be an oral appliance that self titrates based on the desired vs actual efficacy achieved by a patient during a night's sleep.

Other factors known to play a role in appliance therapy comfort include vertical dimension (amount of opening between the upper and lower teeth when appliance is in-situ) which can be increased, decreased, or stabilised by knowing if the patient is excessively opening or moving their lower jaw which may impact the apnea, hypopnea, and snoring. Monitoring these physiological characteristics will allow a subsequent device to be manufactured for a patient that will better treat their symptoms of OSA.

OAT has also been shown to be extremely effective in the treatment of snoring without OSA. Snoring is a common symptom of OSA, but patients may also develop snoring without OSA. Whilst not a medical condition per se, snoring is a common complaint, particularly of sleeping partners.

US 2016/199215, upon which the preamble of claim 1 is based, relates to systems, methods, devices, and apparatus for positioning a patient's mandible in response to sleep apnea status. In one example, the system comprises one or more sensors configured to monitor the patient for symptoms associated with sleep apnea; an intraoral appliance worn by the patient, one or more processors, and memory comprising instructions executable by the one or more processors to cause the one or more processors to: receive a set of sensor data from the one or more sensors, detect, using a machine learning algorithm, onset of a sleep apnea event based on the set of sensor data, and transmit a control signal to the intraoral appliance to cause the intraoral appliance to displace a lower jaw of the patient from a first position to a second position in order to treat the sleep apnea event..

US 2020/197655 relates to a system and method for providing mandibular advancement and sleep positional therapy to a user for a sleep session. US 2017/196727 relates to system and method for improving a sleep disorder, the system including an oral applicance.

Accordingly, there is a need to provide an intra-oral device capable of more accurate monitoring of compliance to and efficacy of OAT for an individual patient.

### Summary

It is an object of the present invention to substantially overcome, or at least ameliorate, one or more disadvantages of existing arrangements.

Disclosed are arrangements which seek to address the above problems by providing an intra-oral device having sensors of sensing biometric information and using the biometric information to determine compliance to and efficacy of OAT for individual patients

According to an aspect of the present disclosure, there is provided an intra-oral device as set out in claim 1.

Another aspect of the present disclosure provides a method of determining efficacy of using an intra-oral device for reducing sleep apnea on a patient as set out in claim 8.

Also provided is a computer program product as set out in claim 15.

### Brief Description of the Drawings

At least one embodiment of the present invention will now be described with reference to the drawings and appendices, in which:
Fig. 1 shows a system for monitoring compliance of OAT and for determining efficacy of the OAT for a patient according to the present disclosure;
Figs. 2A and 2B collectively form a schematic block diagram representation of an intraoral device of the system of Fig. 1;
Fig. 3 shows a schematic block diagram of a docking station of the intra-oral device of Figs. 2A and 2B;
Figs. 4A and 4B form a schematic block diagram of a general purpose computer system upon which the system of Fig. 1 can be implemented;
Fig. 5 is a flow diagram of a method of monitoring compliance of OAT and of determining efficacy of the OAT for a patient according to the present disclosure; and
Figs. 6A, 6B, 7A, and 7B show diagrams of the intra-oral device of Figs. 2A and 2b with a magnetometer and magnet in different configurations.

### Detailed Description

It is to be noted that the discussions contained in the "Background" section and that above relating to prior art arrangements relate to discussions of documents or devices which form public knowledge through their respective publication and/or use. Such should not be interpreted as a representation by the present inventor(s) or the patent applicant that such documents or devices in any way form part of the common general knowledge in the art.

Where reference is made in any one or more of the accompanying drawings to steps and/or features, which have the same reference numerals, those steps and/or features have for the purposes of this description the same function(s) or operation(s), unless the contrary intention appears.

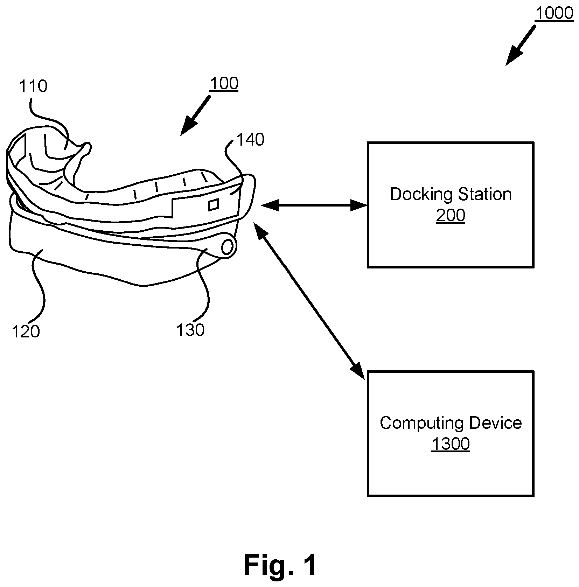

Fig. 1 shows a system 1000 for monitoring compliance of a patient in following OAT and for detecting the efficacy of the OAT on the patient. The system 1000 includes an intra-oral device 100, a docking station 200, and a computing device 1300.

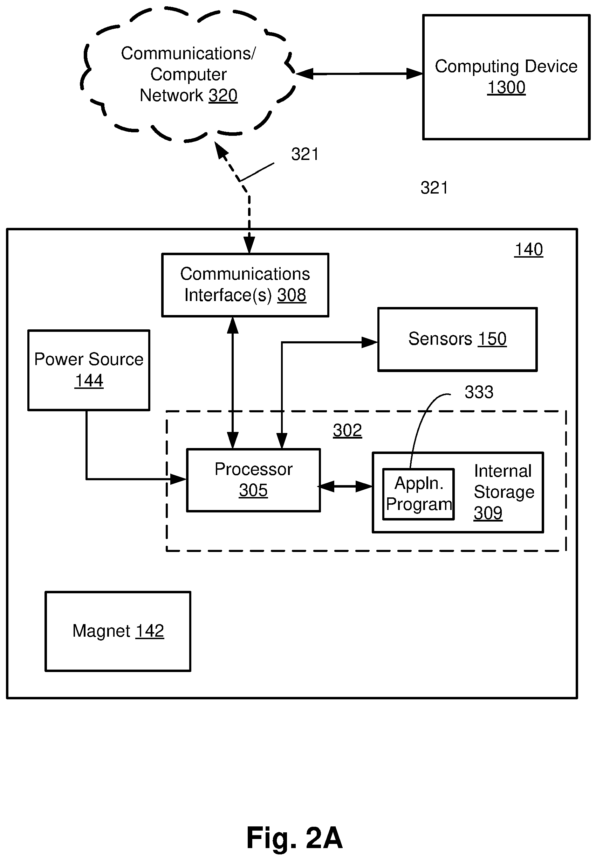

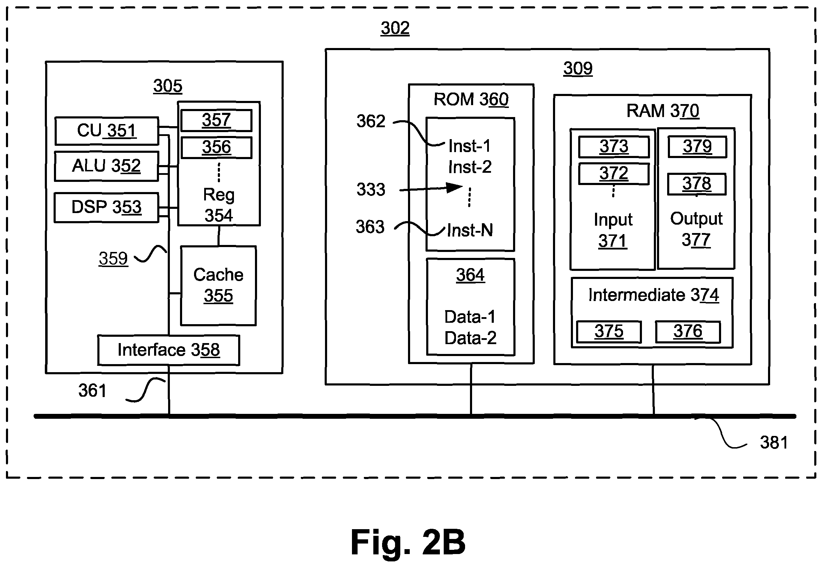

The intra-oral device 100 is a device disposed within the mouth of a patient to reduce sleep apnea and/or snoring. The intra-oral device 100 includes an upper arch component 110, a lower arch component 120, a connector 130, and a sensor assembly 140. The upper arch component 110 and the lower arch component 120 are connected to each other by the connector 130. When in-use, the upper arch component 110 is disposed on the teeth on the upper jaw of the patient, while the lower arch component 120 is disposed on the teeth on the lower jaw of the patient. The connector 130 maintains the position of the lower arch component 120 to be forward relative to the upper arch component 110. This position moves the lower jaw and tongue forward, thereby opening the airway of the patient. The sensor assembly 140 is configured to detect parameters relating to the patient. The sensor assembly 140 will be discussed below in relation to Fig. 2.

In another arrangement, there may be more than one connector 130 used to connect the lower arch component 120 to the upper arch component 110. Examples of the connector 130 are straps, wing/lugs, and telescopic arm.

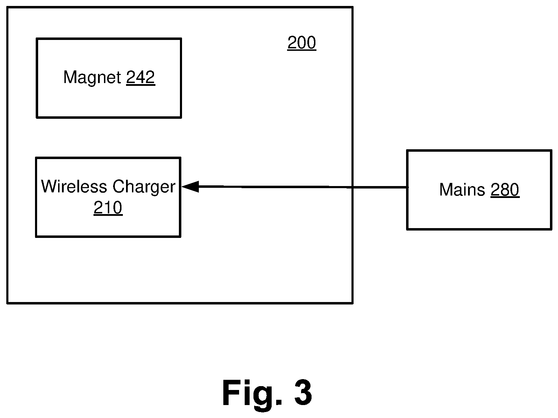

The docking station 200 includes a housing in which the intra-oral device 100 can be disposed. The docking station 200 wirelessly charges the intra-oral device 100, when the intraoral device is docked in the docking station 200. The docking of the intra-oral device 100 within the housing of the docking station 200 will be discussed below in relation to Fig. 3.

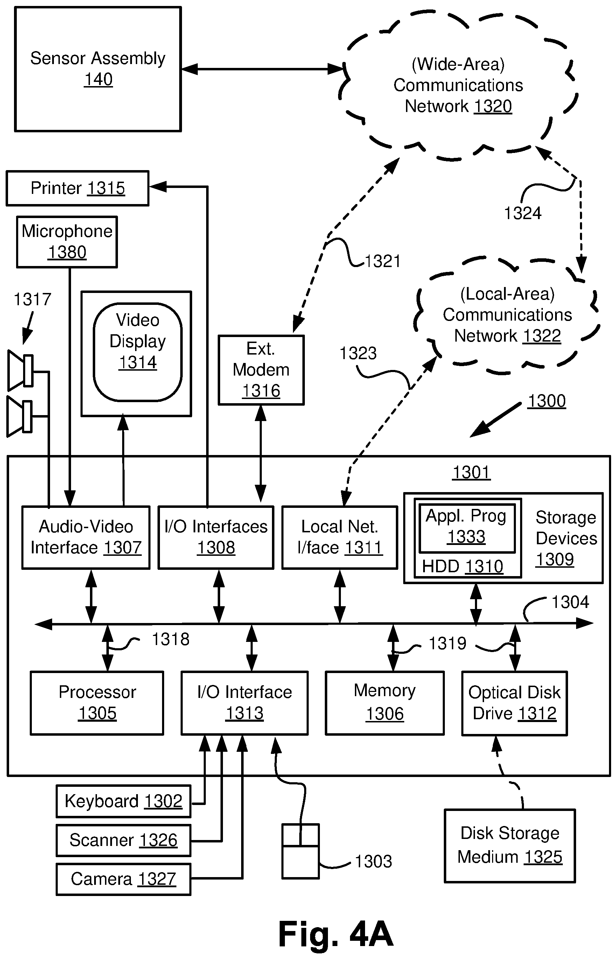

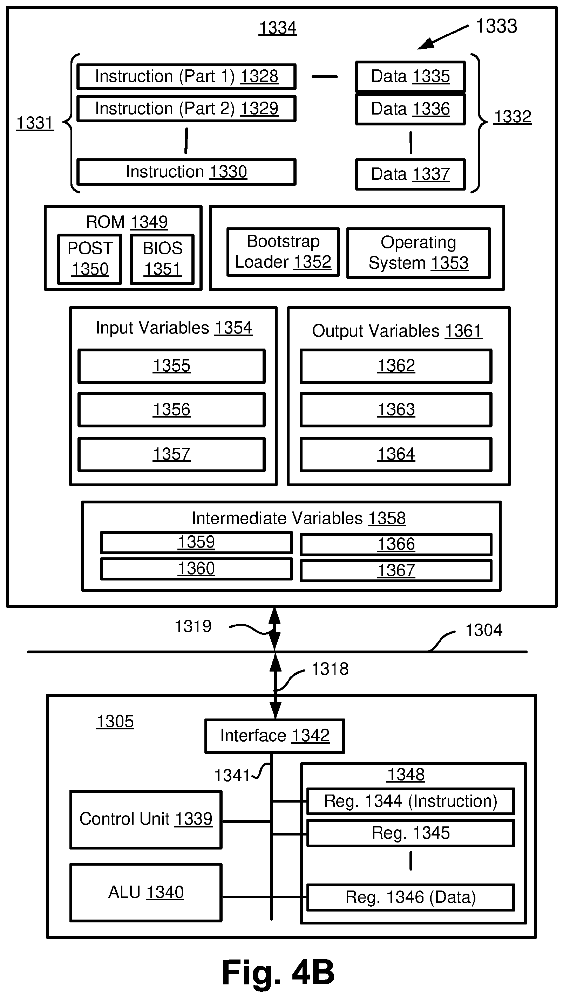

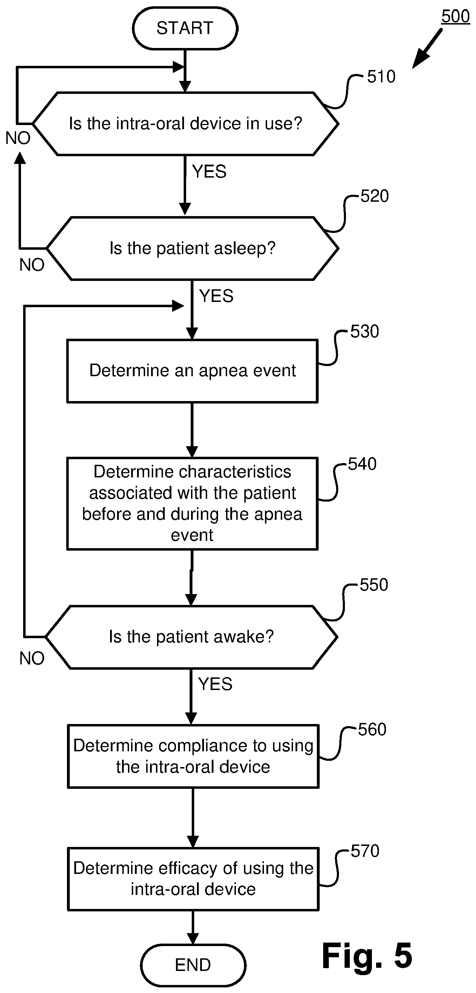

The computing device 1300 is in communication with the sensor assembly 140 of the intra-oral device 100. The computing device 1300 may be a laptop, a desktop computer, a tablet, a smartphone, and the like. The system 1000 provides an app that can be installed on the computing device 1300. The app then analyses the data from the sensor assembly 140 and provides notifications and statistics on a display of the computing device 1300. The app can also forward the data to other computing devices (e.g., a computing device owned by a physician or the patient). The computing device 1300 is described below in relation to Figs. 4A and 4B. The app on the computing device 1300 executes a method of monitoring the compliance of the patient to the OAT and also of detecting the efficacy of the OAT on the patient. The method is described below in relation to Fig. 5.

In an alternative arrangement, the computing device 1300 is in communication with a cloud computing system, which receives the data from the computing device 1300 and analyses the data. The cloud computing system then provides notifications and statistics on a display of the computing device 1300.

### Sensor Assembly 140

Figs. 2A and 2B collectively form a schematic block diagram of the sensor assembly 140 including a power source 144, magnets 142, and sensors 150. The sensors 150 are configured to detect parameters relating to the patient wearing the intra-oral device 100. Although Fig. 1 shows the sensor assembly 140 is shown to be disposed in the upper arch component 110, the sensor assembly 140 may also be disposed in the lower arch component 120.

As seen in Fig. 2A, the sensor assembly 140 also includes an embedded controller 302. The controller 302 has a processing unit (or processor) 305 which is bi-directionally coupled to an internal storage module 309. The storage module 309 may be formed from non-volatile semiconductor read only memory (ROM) 360 and semiconductor random access memory (RAM) 370, as seen in Fig. 3B. The RAM 370 may be volatile, non-volatile or a combination of volatile and non-volatile memory.

The sensor assembly 140 has a communications interface 308 to permit coupling of the sensor assembly 140 to the computing device 1300 or communications network 1320 via a connection 321. The connection 321 is wireless in a preferred arrangement. However, the connection 321 may be wired. For example, the connection 321 may be radio frequency or optical. An example of a wired connection includes Ethernet. Further, an example of wireless connection includes Bluetooth^{™} type local interconnection, Wi-Fi (including protocols based on the standards of the IEEE 802.11 family), Infrared Data Association (IrDa) and the like.

In one arrangement (not shown in Fig. 2A), the sensor assembly 140 couples to one or more external sensors via the communications network 1320. The data collected by the one or more external sensors can then be used to supplement the data collected by the sensor 150.

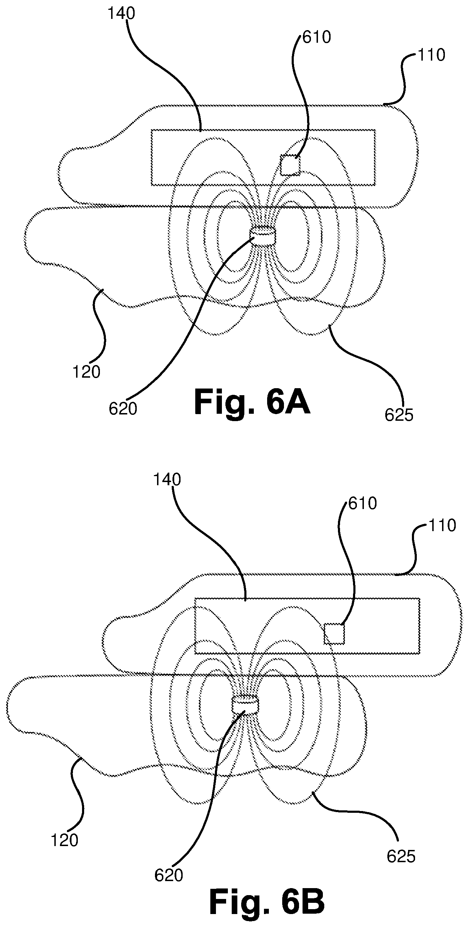

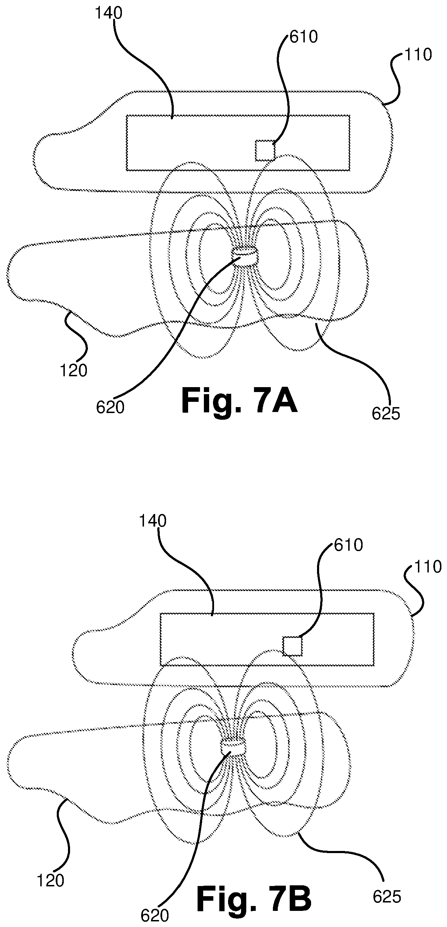

The sensors 150 are configured to detect parameters relating to the patient. In one arrangement, the sensors 150 include a pulse oximeter sensor, an accelerometer, a magnetometer 610 (see Figs. 6A, 6B, 7A, and 7B), a temperature sensor, and a microphone. Other sensors such as a pressure sensor, a switch, and the like may also be included. The embedded controller 302 is in communication with each of the sensors 150 to receive the parameter detected by the sensors 150. In one arrangement, the controller 302 stores the detected parameters in the internal storage 309 and then transmits the detected parameters to the computing device 1300 for analysis. In another arrangement, the controller 302 performs certain steps of the method 500 (see Fig. 5) so that only relevant detected parameters are stored in the internal storage 309, thereby reducing the internal storage 309 requirements.

The pulse oximeter sensor (also known as SpO2 sensor) is a sensor configured to measure the blood oxygen level or the saturation of oxygen in the blood of a patient. The pulse oximeter sensor can also be used to determine the heart rate, heart rate variability and blood pressure of the patient.

The accelerometer sensor is configured to detect the orientation of the sensor assembly 140 and, in turn, the upper arch component 110. As the upper arch component 110 is disposed on the upper jaw of the patient when in use, the orientation of the upper arch component 110 corresponds to the orientation of the head of the patient. Once the head position of the patient is known, the sleeping position of the patient can be determined.

The magnetometer sensor 610 is configured to detect the magnetic field strength of a magnet 620 (see Figs. 6A, 6B, 7A, and 7B). The lower arch component 120 contains the magnet 620, the magnetic field of which is detected by the magnetometer sensor 610. The magnetic field strength of the magnet 620 detected by the magnetometer 610 at different protrusions of the lower arch component 120 can be determined experientially. Further, the magnetic field strength of the magnet 620 detected by the magnetometer 610 when the upper arch component 110 is apart from the lower arch component 120 can be determined experientially. Based on the experiment data, the protrusion of the lower arch component 120 and how far apart the upper arch component 110 is from the lower arch component 120 (e.g., whether a mouth is open, shifted to one side or protruded) can be determined.

Figs. 6A, 6B, 7A, and 7B show the magnet 620 in communication with the magnetometer 610 (which is one of the sensors 150 disposed in the sensor assembly 140). The communication between the magnet 620 and the magnetometer 610 is in the form of the magnetometer 610 detecting the magnetic field 625 generated by the magnet 620. The magnet 620 is disposed in the arch component opposite to the arch component in which the sensor assembly 140 is disposed. In other words, if the sensor assembly 140 is disposed in the upper arch component 110, then the magnet 620 is disposed in the lower arch component 120. If the sensor assembly 140 is disposed in the lower arch component 120, then the magnet 620 is disposed in the upper arch component 110.

Figs. 6A and 7A show two examples of the intra-oral device 100 in a first configuration. The first configuration is when a user places the intra-oral device 100 in the mouth before advancing the lower arch component 120. Depending on the jaw structure of the user, the intra-oral device 100 may be in a first configuration shown in either Fig. 6A or 7A. There are also other possible configurations not shown.

Figs 6B and 7B show two examples of the intra-oral device 100 in a second configuration. The second configuration is when the intra-oral device 100 is in operation such that the lower arch component 120 is moved forward relative to the upper arch component 110. The second configuration of Fig. 6B corresponds to the first configuration shown in Fig. 6A. Similarly, the second configuration of Fig. 7B corresponds to the first configuration shown in Fig. 7A.

As discussed above, the strength of the magnetic field 625 when the magnet 620 is located at different positions relative to the magnet 610 is determined experientially. In the first configuration, the detected strength of the magnetic field 625 is recorded and the position corresponding to the detected strength of the magnetic field 625 is set to be the position of the first configuration. Recording the position in the first configuration can be performed during a setup stage. The setup stage may be performed by a physician administering the OAT or by the user.

In the second configuration, the detected strength of the magnetic field 625 is recorded and the position corresponding to the detected strength of the magnetic field 625 is set to be the position of the second configuration. The advancement of the lower arch component 120 relative to the upper arch component 110 can then be determined by comparing the position of the first configuration against the position of the second configuration.

The above enables the determination of the advancement of the lower arch component 120 relative to the upper arch component 110 to be customized for each user. In tum, such customization enables a more accurate determination of the advancement. Any changes to the typical advancement determination of a user may also be used to determine issues with the OTA (e.g., whether the user has not complied with the prescribed OAT).

The temperature sensor is configured to detect a change of temperature. An increase in temperature to a normal bodily temperature can be used as an indicator that the intra-oral device 110 is disposed in the mouth.

The microphone is configured to detect sound. The controller 302 or the computing device 1300 can then analyse the detected sound to determine breathing activity (e.g., normal breathing, snoring patterns, bruxing etc.).

Other sensors such as a pressure sensor or a switch may be included to determine whether the intra-oral device is in use or determine the pressure of a bite (e.g., for detecting bruxism).

The sensor assembly 140 also includes a power source 144 and a magnet 142. The power source 144 includes a wireless power receiver, a battery, and a voltage regulator. The components of the power source 144 are not shown in Fig. 2A for simplicity sake. The wireless power receiver is configured to receive power wirelessly in order to charge the battery. The battery is configured to provide power to the voltage regulator, which in turn provides the regulated power to the controller 302.

The magnet 142 is used to engage a corresponding magnet 242 located in the docking station 200. When the magnets 142 and 242 are engaged, the wireless power receiver of the power source 144 is aligned with the transmitter coil of the wireless charger 210 located at the docking station 200.

The methods described hereinafter may be implemented using the embedded controller 302, where steps 510, 520, and 550 of Fig. 5 may be implemented as one or more software application programs 333 executable within the embedded controller 302. In particular, with reference to Fig. 3B, steps 510, 520, and 550 of the described method 500 are effected by instructions in the software 333 that are carried out within the controller 302. The software instructions may be formed as one or more code modules, each for performing one or more particular tasks.

The software 333 of the embedded controller 302 is typically stored in the non-volatile ROM 360 of the internal storage module 309. The software 333 stored in the ROM 360 can be updated when required from a computer readable medium. The software 333 can be loaded into and executed by the processor 305. In some instances, the processor 305 may execute software instructions that are located in RAM 370. Software instructions may be loaded into the RAM 370 by the processor 305 initiating a copy of one or more code modules from ROM 360 into RAM 370. Alternatively, the software instructions of one or more code modules may be pre-installed in a non-volatile region of RAM 370 by a manufacturer. After one or more code modules have been located in RAM 370, the processor 305 may execute software instructions of the one or more code modules.

The application program 333 is typically pre-installed and stored in the ROM 360 by a manufacturer, prior to installation of the sensor assembly 140 in the upper arch component 110. However, in some instances, the application programs 333 may be read by the processor 305 from the network 1320. Computer readable storage media refers to any non-transitory tangible storage medium that participates in providing instructions and/or data to the controller 302 for execution and/or processing. Examples of such storage media include a solid state drive, a ROM or integrated circuit, USB memory, and the like. Examples of transitory or non-tangible computer readable transmission media that may also participate in the provision of software, application programs, instructions and/or data to the sensor assembly 140 include radio or infrared transmission channels as well as a network connection to another computer or networked device, and the Internet or Intranets including e-mail transmissions and information recorded on Websites and the like. A computer readable medium having such software or computer program recorded on it is a computer program product.

Fig. 2B illustrates in detail the embedded controller 302 having the processor 305 for executing the application programs 333 and the internal storage 309. The internal storage 309 comprises read only memory (ROM) 360 and random access memory (RAM) 370. The processor 305 is able to execute the application programs 333 stored in one or both of the connected memories 360 and 370. When the sensor assembly 140 is initially powered up, a system program resident in the ROM 360 is executed. The application program 333 permanently stored in the ROM 360 is sometimes referred to as "firmware". Execution of the firmware by the processor 305 may fulfil various functions, including processor management, memory management, device management, storage management and user interface.

The processor 305 typically includes a number of functional modules including a control unit (CU) 351, an arithmetic logic unit (ALU) 352, a digital signal processor (DSP) 353 and a local or internal memory comprising a set of registers 354 which typically contain atomic data elements 356, 357, along with internal buffer or cache memory 355. One or more internal buses 359 interconnect these functional modules. The processor 305 typically also has one or more interfaces 358 for communicating with external devices via system bus 381, using a connection 361.

The application program 333 includes a sequence of instructions 362 through 363 that may include conditional branch and loop instructions. The program 333 may also include data, which is used in execution of the program 333. This data may be stored as part of the instruction or in a separate location 364 within the ROM 360 or RAM 370.

In general, the processor 305 is given a set of instructions, which are executed therein. This set of instructions may be organised into blocks, which perform specific tasks or handle specific events that occur in the sensor assembly 140. Typically, the application program 333 waits for events and subsequently executes the block of code associated with that event. Events may be triggered in response to input from a user, via the user input devices 313 of Fig. 3A, as detected by the processor 305. Events may also be triggered in response to other sensors and interfaces in the sensor assembly 140.

The execution of a set of the instructions may require numeric variables to be read and modified. Such numeric variables are stored in the RAM 370. The disclosed method uses input variables 371 that are stored in known locations 372, 373 in the memory 370. The input variables 371 are processed to produce output variables 377 that are stored in known locations 378, 379 in the memory 370. Intermediate variables 374 may be stored in additional memory locations in locations 375, 376 of the memory 370. Alternatively, some intermediate variables may only exist in the registers 354 of the processor 305.

The execution of a sequence of instructions is achieved in the processor 305 by repeated application of a fetch-execute cycle. The control unit 351 of the processor 305 maintains a register called the program counter, which contains the address in ROM 360 or RAM 370 of the next instruction to be executed. At the start of the fetch execute cycle, the contents of the memory address indexed by the program counter is loaded into the control unit 351. The instruction thus loaded controls the subsequent operation of the processor 305, causing for example, data to be loaded from ROM memory 360 into processor registers 354, the contents of a register to be arithmetically combined with the contents of another register, the contents of a register to be written to the location stored in another register and so on. At the end of the fetch execute cycle the program counter is updated to point to the next instruction in the system program code. Depending on the instruction just executed this may involve incrementing the address contained in the program counter or loading the program counter with a new address in order to achieve a branch operation.

Each step 510, 520, or 550 of the method 500 described below is associated with one or more segments of the application program 333, and is performed by repeated execution of a fetch-execute cycle in the processor 305 or similar programmatic operation of other independent processor blocks in the electronic device 301.

### Docking Station 200

Fig. 3 shows a schematic of the docking station 200. The docking station 200 includes a wireless charger 210 and a magnet 242. As described above in relation to the sensor assembly 140, the magnet 242 is configured to engage with the magnet 142 disposed in the sensor assembly 140. When the magnets 142 and 242 are engaged, the transmitter coil of the wireless charger 210 is aligned with the wireless power receiver in the sensor assembly 140. The alignment of the transmitter coil of the wireless charger 210 and the wireless power receiver enables power to be supplied from the wireless charger 210 to the battery of the power source 144.

The wireless charger 210 receives electrical power from the mains 280. There is electronic circuitry (not shown or discussed here for simplicity sake) configured to convert the AC electrical power from the mains 280 to electrical power suitable for charging the power source 144 by the wireless charger 210.

The docking station 200 also includes a housing into which the intra-oral device 100 can be disposed.

### Computing Device 1300

Figs. 4A and 4B depict the computing device 1300. As seen in Fig. 4A, the computing device 1300 includes: a computer module 1301; input devices such as a keyboard 1302, a mouse pointer device 1303, a scanner 1326, a camera 1327, and a microphone 1380; and output devices including a printer 1315, a display device 1314 and loudspeakers 1317. An external Modulator-Demodulator (Modem) transceiver device 1316 may be used by the computer module 1301 for communicating to and from a communications network 1320 via a connection 1321. The communications network 1320 may be a wide-area network (WAN), such as the Internet, a cellular telecommunications network, or a private WAN. Where the connection 1321 is a telephone line, the modem 1316 may be a traditional "dial-up" modem. Alternatively, where the connection 1321 is a high capacity (e.g., cable) connection, the modem 1316 may be a broadband modem. A wireless modem may also be used for wireless connection to the communications network 1320.

The computer module 1301 typically includes at least one processor unit 1305, and a memory unit 1306. For example, the memory unit 1306 may have semiconductor random access memory (RAM) and semiconductor read only memory (ROM). The computer module 1301 also includes an number of input/output (I/O) interfaces including: an audio-video interface 1307 that couples to the video display 1314, loudspeakers 1317 and microphone 1380; an I/O interface 1313 that couples to the keyboard 1302, mouse 1303, scanner 1326, camera 1327 and optionally a joystick or other human interface device (not illustrated); and an interface 1308 for the external modem 1316 and printer 1315. In some implementations, the modem 1316 may be incorporated within the computer module 1301, for example within the interface 1308. The computer module 1301 also has a local network interface 1311, which permits coupling of the computing device 1300 via a connection 1323 to a local-area communications network 1322, known as a Local Area Network (LAN). As illustrated in Fig. 4A, the local communications network 1322 may also couple to the wide network 1320 via a connection 1324, which would typically include a so-called "firewall" device or device of similar functionality. The local network interface 1311 may comprise an Ethernet circuit card, a Bluetooth^{®} wireless arrangement or an IEEE 802.11 wireless arrangement; however, numerous other types of interfaces may be practiced for the interface 1311.

The I/O interfaces 1308 and 1313 may afford either or both of serial and parallel connectivity, the former typically being implemented according to the Universal Serial Bus (USB) standards and having corresponding USB connectors (not illustrated). Storage devices 1309 are provided and typically include a hard disk drive (HDD) 1310. Other storage devices such as a floppy disk drive and a magnetic tape drive (not illustrated) may also be used. An optical disk drive 1312 is typically provided to act as a non-volatile source of data. Portable memory devices, such optical disks (e.g., CD-ROM, DVD, Blu-ray Disc^{™}), USB-RAM, portable, external hard drives, and floppy disks, for example, may be used as appropriate sources of data to the computing device 1300.

The components 1305 to 1313 of the computer module 1301 typically communicate via an interconnected bus 1304 and in a manner that results in a conventional mode of operation of the computing device 1300 known to those in the relevant art. For example, the processor 1305 is coupled to the system bus 1304 using a connection 1318. Likewise, the memory 1306 and optical disk drive 1312 are coupled to the system bus 1304 by connections 1319. Examples of computers on which the described arrangements can be practised include IBM-PC's and compatibles, Sun SPARCstations, Apple Mac^{™} or like computer systems.

The method of determining compliance to and efficacy of using the intra-oral device 100 may be implemented using the computing device 1300 wherein the processes of Fig. 5, to be described, may be implemented as one or more software application programs 1333 executable within the computing device 1300. In particular, the steps of the method of determining compliance to and efficacy of using the intra-oral device 100 are effected by instructions 1331 (see Fig. 4B) in the software 1333 that are carried out within the computing device 1300. The software instructions 1331 may be formed as one or more code modules, each for performing one or more particular tasks. The software may also be divided into two separate parts, in which a first part and the corresponding code modules performs the determining compliance to and efficacy of using the intra-oral device 100 methods and a second part and the corresponding code modules manage a user interface between the first part and the user.

The software may be stored in a computer readable medium, including the storage devices described below, for example. The software is loaded into the computing device 1300 from the computer readable medium, and then executed by the computing device 1300. A computer readable medium having such software or computer program recorded on the computer readable medium is a computer program product. The use of the computer program product in the computing device 1300 preferably effects an advantageous apparatus for determining compliance to and efficacy of using the intra-oral device 100.

The software 1333 is typically stored in the HDD 1310 or the memory 1306. The software is loaded into the computing device 1300 from a computer readable medium, and executed by the computing device 1300. Thus, for example, the software 1333 may be stored on an optically readable disk storage medium (e.g., CD-ROM) 1325 that is read by the optical disk drive 1312. A computer readable medium having such software or computer program recorded on it is a computer program product. The use of the computer program product in the computing device 1300 preferably effects an apparatus for determining compliance to and efficacy of using the intra-oral device 100.

In some instances, the application programs 1333 may be supplied to the user encoded on one or more CD-ROMs 1325 and read via the corresponding drive 1312, or alternatively may be read by the user from the networks 1320 or 1322. Still further, the software can also be loaded into the computing device 1300 from other computer readable media. Computer readable storage media refers to any non-transitory tangible storage medium that provides recorded instructions and/or data to the computing device 1300 for execution and/or processing. Examples of such storage media include floppy disks, magnetic tape, CD-ROM, DVD, Bluray^{™} Disc, a hard disk drive, a ROM or integrated circuit, USB memory, a magneto-optical disk, or a computer readable card such as a PCMCIA card and the like, whether or not such devices are internal or external of the computer module 1301. Examples of transitory or non-tangible computer readable transmission media that may also participate in the provision of software, application programs, instructions and/or data to the computer module 1301 include radio or infra-red transmission channels as well as a network connection to another computer or networked device, and the Internet or Intranets including e-mail transmissions and information recorded on Websites and the like.

The second part of the application programs 1333 and the corresponding code modules mentioned above may be executed to implement one or more graphical user interfaces (GUIs) to be rendered or otherwise represented upon the display 1314. Through manipulation of typically the keyboard 1302 and the mouse 1303, a user of the computing device 1300 and the application may manipulate the interface in a functionally adaptable manner to provide controlling commands and/or input to the applications associated with the GUI(s). Other forms of functionally adaptable user interfaces may also be implemented, such as an audio interface utilizing speech prompts output via the loudspeakers 1317 and user voice commands input via the microphone 1380.

Fig. 4B is a detailed schematic block diagram of the processor 1305 and a "memory" 1334. The memory 1334 represents a logical aggregation of all the memory modules (including the HDD 1309 and semiconductor memory 1306) that can be accessed by the computer module 1301 in Fig. 4A.

When the computer module 1301 is initially powered up, a power-on self-test (POST) program 1350 executes. The POST program 1350 is typically stored in a ROM 1349 of the semiconductor memory 1306 of Fig. 4A. A hardware device such as the ROM 1349 storing software is sometimes referred to as firmware. The POST program 1350 examines hardware within the computer module 1301 to ensure proper functioning and typically checks the processor 1305, the memory 1334 (1309, 1306), and a basic input-output systems software (BIOS) module 1351, also typically stored in the ROM 1349, for correct operation. Once the POST program 1350 has run successfully, the BIOS 1351 activates the hard disk drive 1310 of Fig. 4A. Activation of the hard disk drive 1310 causes a bootstrap loader program 1352 that is resident on the hard disk drive 1310 to execute via the processor 1305. This loads an operating system 1353 into the RAM memory 1306, upon which the operating system 1353 commences operation. The operating system 1353 is a system level application, executable by the processor 1305, to fulfil various high level functions, including processor management, memory management, device management, storage management, software application interface, and generic user interface.

The operating system 1353 manages the memory 1334 (1309, 1306) to ensure that each process or application running on the computer module 1301 has sufficient memory in which to execute without colliding with memory allocated to another process. Furthermore, the different types of memory available in the computing device 1300 of Fig. 4A must be used properly so that each process can run effectively. Accordingly, the aggregated memory 1334 is not intended to illustrate how particular segments of memory are allocated (unless otherwise stated), but rather to provide a general view of the memory accessible by the computing device 1300 and how such is used.

As shown in Fig. 4B, the processor 1305 includes a number of functional modules including a control unit 1339, an arithmetic logic unit (ALU) 1340, and a local or internal memory 1348, sometimes called a cache memory. The cache memory 1348 typically includes a number of storage registers 1344 - 1346 in a register section. One or more internal busses 1341 functionally interconnect these functional modules. The processor 1305 typically also has one or more interfaces 1342 for communicating with external devices via the system bus 1304, using a connection 1318. The memory 1334 is coupled to the bus 1304 using a connection 1319.

The application program 1333 includes a sequence of instructions 1331 that may include conditional branch and loop instructions. The program 1333 may also include data 1332 which is used in execution of the program 1333. The instructions 1331 and the data 1332 are stored in memory locations 1328, 1329, 1330 and 1335, 1336, 1337, respectively. Depending upon the relative size of the instructions 1331 and the memory locations 1328-1330, a particular instruction may be stored in a single memory location as depicted by the instruction shown in the memory location 1330. Alternately, an instruction may be segmented into a number of parts each of which is stored in a separate memory location, as depicted by the instruction segments shown in the memory locations 1328 and 1329.

In general, the processor 1305 is given a set of instructions which are executed therein. The processor 1305 waits for a subsequent input, to which the processor 1305 reacts to by executing another set of instructions. Each input may be provided from one or more of a number of sources, including data generated by one or more of the input devices 1302, 1303, data received from an external source across one of the networks 1320, 1302, data retrieved from one of the storage devices 1306, 1309 or data retrieved from a storage medium 1325 inserted into the corresponding reader 1312, all depicted in Fig. 4A. The execution of a set of the instructions may in some cases result in output of data. Execution may also involve storing data or variables to the memory 1334.

The disclosed method of determining compliance to and efficacy of using the intra-oral device 100 arrangements use input variables 1354, which are stored in the memory 1334 in corresponding memory locations 1355, 1356, 1357. The disclosed arrangements produce output variables 1361, which are stored in the memory 1334 in corresponding memory locations 1362, 1363, 1364. Intermediate variables 1358 may be stored in memory locations 1359, 1360, 1366 and 1367.

Referring to the processor 1305 of Fig. 4B, the registers 1344, 1345, 1346, the arithmetic logic unit (ALU) 1340, and the control unit 1339 work together to perform sequences of micro-operations needed to perform "fetch, decode, and execute" cycles for every instruction in the instruction set making up the program 1333. Each fetch, decode, and execute cycle comprises:
a fetch operation, which fetches or reads an instruction 1331 from a memory location 1328, 1329, 1330;
a decode operation in which the control unit 1339 determines which instruction has been fetched; and
an execute operation in which the control unit 1339 and/or the ALU 1340 execute the instruction.

Thereafter, a further fetch, decode, and execute cycle for the next instruction may be executed. Similarly, a store cycle may be performed by which the control unit 1339 stores or writes a value to a memory location 1332.

Each step or sub-process in the processes of Fig. 5 is associated with one or more segments of the program 1333 and is performed by the register section 1344, 1345, 1347, the ALU 1340, and the control unit 1339 in the processor 1305 working together to perform the fetch, decode, and execute cycles for every instruction in the instruction set for the noted segments of the program 1333.

The method of determining compliance to and efficacy of using the intra-oral device 100 may alternatively be implemented in dedicated hardware such as one or more integrated circuits performing the functions or sub functions of the method 500. Such dedicated hardware may include graphic processors, digital signal processors, or one or more microprocessors and associated memories. Such a dedicated hardware may be disposed at the docking station 200 or the sensor assembly 140.

### Method 500

Fig. 5 shows a method 500 of determining compliance to and efficacy of using the intraoral device 100. The method 500 is computer program, the steps of which are executable by the processor 305 or 1305.

The method 500 commences with an optional step 510 by determining whether the intra-oral device 100 is in use. As mentioned hereinbefore, step 510 may be implemented by the sensor assembly 140 before executing the further steps of the method 500. The optional step 510 enables the sensor assembly 140 to record data when the patient is wearing the intra-oral device 100.

Step 510 can also be performed by the controller 1305. If the controller 1305 performs step 510, the controller 305 records all the data detected by the sensors 150. The controller 1305 then analyses the recorded data.

In one arrangement, the method 500 commences when the intra-oral device 100 is removed from the docking station 200. In an alternative arrangement, the method 500 is executed continually. If step 510 is not implemented, the method 500 commences from step 520.

There are multiple characteristics that can be used for determining whether the intra-oral device 100 is in use. These characteristics include the temperature, blood pressure, heart rate, and blood oxygen level. The characteristics for determining whether the intra-oral device is in use will be hereinafter referred to as the use characteristics.

The temperature sensor in the sensor assembly 140 is used to determine a change in temperature. When the detected temperature is in the range of 35°C to 41°C (i.e., a body temperature), the controller 305 or 1305 determines that the intra-oral device 100 to be in use by the patient.

The pulse oximeter sensor is used to determine a blood oxygen level, a blood pressure, and heart rate of a patient. If the pulse oximeter sensor detects a blood oxygen level of above a predetermined threshold (e.g., 90%), then the controller 305 or 1305 determines the intra-oral device 100 to be in use. Further, when a heart rate is detected, the controller 305 or 1305 determines that the intra-oral device 100 to be in use. Further, when a blood pressure is detected, the controller 305 or 1305 determines that the intra-oral device 100 is in use.

Combinations of the use characteristics can also be used in step 510 to more accurately determine when the intra-oral device 100 is in use. For example, if the temperature sensor is within the range of 35°C to 41°C but no heart rate is detected, the method 500 determines that the intra-oral device 100 is not in use.

If the intra-oral device is determined to be in use (YES), then the method 500 proceeds from step 510 to step 520. Otherwise (NO), the method 500 remains at step 510 to continually determine whether the intra-oral device 100 is in use. This is recorded as usage time. Usage time stops when the device is removed from the mouth and no signals are recorded.

Step 520 determines whether the patient is asleep. As mentioned hereinbefore, step 520 may be implemented by the sensor assembly 140 before executing the further steps of the method 500. Step 520 enables the sensor assembly 140 to record data when the patient is asleep so that only relevant data is recorded. In another arrangement, the sensor assembly 140 records data at all time. If this other arrangement is implemented, steps 510 and 520 can occur simultaneously with step 530.

Step 520 can also be performed by the controller 1305. If the controller 1305 performs step 520, the controller 305 records all the data detected by the sensors 150. The controller 1305 then analyses the recorded data.

There are multiple characteristics that can be used for determining whether a patient is asleep. These characteristics include pulse oximetry, pulse wave, detection of snoring sounds, mandibular movement and heart rate. The characteristics for determining whether the patient is asleep will be hereinafter referred to as the sleep characteristics.

The pulse oximeter sensor is used to determine the pulse wave, blood pressure and heart rate of the patient. When the detected blood pressure drops by 20-50mmHg, or the height of the pulse wave reduces by 20% or more, the controller 305 determines that the patient is asleep. When the detected heart rate drops by 10-20 beats/ minute the controller 305 determines that the patient is asleep.

The sleep characteristics can be customised to a patient based on the recorded data. For example, the default for determining when a patient falls asleep is when the blood pressure drops by 20-50mmHg. However, over time, the computer software being executed by the controller 305 or 1305 may obtain more data to better determine when a particular patient's blood pressure when falling asleep. The method 500 then adjust the predetermined threshold so that the method 500 can better determine when that particular patient falls asleep.

A combination of the sleep characteristics can also be used in step 520 to more accurately determine when the patient is asleep. For example, if the blood pressure drops below the predetermined threshold but the heart rate is still high, the method 500 determines that the patient is not asleep yet.

When the method 500 determines that the patient is asleep, the method 500 logs the time that the patient is determined to be asleep. This is recorded as sleep time and stops when the patient is detected to be awake.

If the patient is determined to be asleep (YES), then the method 500 proceeds from step 520 to step 530. Otherwise (NO), the method 500 returns to step 510 to determine whether the intra-oral device 110 is still in use.

Step 530 determines an apnea or hypopnea event. There are multiple characteristics that can be used as indicators for determining an apnea event. These characteristics include blood oxygen level, heart rate, mandibular and head positions, and certain breathing activity (e.g., gasping for air). The characteristics for determining an apnea event will be hereinafter referred to as the apnea characteristics. Step 530 is performed by the processor 1305.

The pulse oximeter sensor is used to determine the blood oxygen level and heart rate of the patient. When the detected blood oxygen level drops by greater than or equal to 3% and, at around the same time, the heart rate increases by 10-50% the controller 1305 determines that an apnea event occurs. The drop in blood oxygen level and increase in heart rate are used as the primary indicator as to whether an apnea event occurs.

The mandibular position is determined by using the magnetic field strength determined by the magnetometer. As discussed hereinbefore, the magnetic field strength determined by the magnetometer in the upper arch component 110 indicates the protrusion of the lower arch component 120 and also how far apart the lower arch component 120 is from the upper arch component 110. When the mouth is determined to be opening after detecting a drop in blood pressure and an increase in heart rate, this is an indicator of an apnea event occurring. The opening of the mouth is used as a secondary indicator whether an apnea event occurs.

The gasping for air of a patient can be determined by analysing the sound recorded by the microphone. The gasping for air is an increase in sound amplitude for a short period of time. The gasp for air occurs simultaneously with the opening of the mouth and therefore a gasp for air is used as a secondary indicator whether an apnea event occurs.

The apnea characteristics can be customised to a patient based on the recorded data. For example, the default for determining when an apnea event occurs is when the blood oxygen level drops by greater than or equal to 3%. However, over time, the computer software being executed by the controller 1305 obtains more data to better determine when a particular patient's apnea event occurs. The method 500 then adjusts the predetermined threshold so that the method 500 can better determine when an apnea event occurs.

The method 500 then proceeds from step 530 to step 540.

Step 540 determines characteristics associated with the patient before and during the apnea event. Step 540 is performed by the processor 1305 and can be executed at any time after step 520 concludes.

There are multiple characteristics recorded to perform step 540. These characteristics will be hereinafter referred to as the patient characteristics. The patient characteristics include the position of the head and mandible of the patient, the protrusion of the lower arch component 120 in relation to the upper arch component 110, breathing activity, oxygen saturation and acceleration of the patient's head and jaw.

The position of the head of the patient can be determined based on the data recorded by the accelerometer. For example, if the accelerometer indicates that accelerometer is oriented vertically upward, then such provides an indication that the head is facing upward and that the patient is sleeping in a supine position. In another example, if the accelerometer indicates that the accelerometer is oriented horizontally, then such provides an indication that the head is facing sideway and that the patient is sleeping in a side position.

The protrusion of the lower arch component 120 can be determined based on the data recorded by the magnetometer. As discussed hereinbefore, the protrusion of the lower arch component 120 and how far apart the upper arch component 110 is from the lower arch component 120 (e.g., whether a mouth is open, closed or shifted to one side) can be determined by using experimental and recorded data.

The breathing activity of the patient can be determined by analysing the sound recorded by the microphone. The controller 1305 executes a software program analysing the frequency and periodical pattern of the patient's breathing to determine whether the patient is breathing normally, snoring, gasping, and the like.

The method 500 proceeds to step 550 at the conclusion of step 540.

Step 550 determines whether the patient is awake. As mentioned hereinbefore, step 550 may be implemented by the sensor assembly 140. Step 550 enables the sensor assembly 140 to stop recording data when the patient is determined to be awake. In one arrangement, recording of data is not stopped to enable the recorded data to be analysed shortly after the patient is awake.

Step 550 can also be performed by the controller 1305. If the controller 1305 performs step 550, the controller 305 records all the data detected by the sensors 150. The controller 1305 then analyses the recorded data.

The sleep characteristics are used for determining whether a patient is awake.

The pulse oximeter sensor is used to determine the blood pressure and heart rate of the patient. When the detected blood pressure increases 20-50mmHg, the controller 305 determines that the patient is awake. When the detected heart rate increases 10-50%, the controller 305 determines that the patient is awake.

The sleep characteristics can be customised to a patient based on the recorded data. For example, the default for determining when a patient wakes up is when the blood pressure increases by 20-50mmHg. However, over time, the computer software being executed by the controller 305 or 1305 obtains more data to better determine when a particular patient's blood pressure when waking up. The method 500 then adjusts the predetermined threshold so that the method 500 can better determine when that particular patient wakes up.

A combination of the sleep characteristics can also be used in step 550 to more accurately determine when the patient is awake. For example, if the blood pressure/ pulse wave increases above the predetermined threshold but the heart rate is still low, the method 500 determines that the patient is not awake yet.

When the method 500 determines that the patient is awake, the method 500 logs the time that the patient is determined to be awake.

If the patient is determined to be awake (YES), then the method 500 proceeds from step 550 to step 560. Otherwise (NO), the method 500 returns to step 530 to continually determine apnea events and the patient characteristics.

Step 560 determines the compliance of the patient to a prescribed OAT using the intraoral device 100. Step 560 is performed by the controller 1305.

Step 560 is performed by comparing the recorded usage of the intra-oral device 100 with the prescribed amount of using the intra-oral device 100 during sleep. The recorded usage of the intra-oral device 100 is determined from the logged times of the patient being asleep and awake. This recorded usage is then compared against the prescribed usage time. If the recorded usage falls below the prescribed usage time, then the method 500 outputs a compliance percentage of the patient.

The method 500 then proceeds from step 560 to step 570.

Step 570 determines the efficacy of the intra-oral device 100 in reducing the apnea events occurring during sleep. Step 570 is performed by the controller 1305.

Step 570 is performed by determining AHI of the patient. AHI is determined by comparing the number of apnea events occurring every hour the patient is asleep. The efficacy of the OAT using the intra-oral device 100 can be better determined using the patient characteristics. For example, the mandibular and head position of the patient, breathing activity (e.g., snoring), and the like when apnea events occur may provide a better insight into what is causing the apnea event and whether the intra-oral device 100 assists in reducing the number of apnea events occurring.

The method 500 concludes at the conclusion of step 570.

Based on the data collected by the sensor assembly 140, a physician uses the data to improve the OAT (e.g., by increasing the protrusion, changing the intra-oral device, increasing titration, adding a supplemental therapy, etc.).

### Industrial Applicability

The arrangements described are applicable to the computer and data processing industries and particularly for determining the compliance to and efficacy of using the intra-oral device 100.

The foregoing describes only some embodiments of the present invention, and modifications and/or changes can be made thereto without departing from the scope of the invention, the embodiments being illustrative and not restrictive.

In the context of this specification, the word "comprising" means "including principally but not necessarily solely" or "having" or "including", and not "consisting only of". Variations of the word "comprising", such as "comprise" and "comprises" have correspondingly varied meanings.

## Claims

1. An intra-oral device (100) comprising:
a lower arch component (120) and an upper arch component (110), wherein the lower arch component (120) is configured to be advanced relative to the upper arch component (120);
sensors (150) configured for detecting characteristics of a patient,
wherein the sensors (150) are disposed in the upper arch component (110) or the lower arch component (120), wherein the characteristics comprise indicators of sleep apnea on the patient and the advancement of the lower arch component (120) relative to the upper arch component (110); and
a processor (305) in communication with the sensors (150) to receive the detected characteristics,
**characterized in that**:
the sensors (150) comprise a magnetometer (610) configured to detect a magnetic field generated by a magnet (620) disposed in the opposite arch component; and
further wherein the processor (305) is configured to:
determine, based on the detected magnetic field, the advancement of the lower arch component (120) relative to the upper arch component (110); and
determine, based on the detected characteristics and the determined advancement, the efficacy of the intra-oral device (100) in reducing the sleep apnea of the patient.

2. The intra-oral device (100) of claim 1, wherein the sensors (150) comprise a pulse oximeter sensor, an accelerometer, a temperature sensor, and a microphone.

3. The intra-oral device (100) of claim 1 or 2, wherein the processor (305) is further configured to determine, based on the detected characteristics compliance of the patient to using the intra-oral device (100) according to a prescribed oral appliance therapy.

4. The intra-oral device (100) of any one of claims 1 to 3, wherein the detected characteristics include use characteristics comprising a temperature, a blood pressure, a heart rate, and a blood oxygen level of the patient, and wherein the processor (305) is further configured to determine, based on the use characteristics, whether the intra-oral device (100) is being used by the patient.

5. The intra-oral device (100) of any one of claims 1 to 4, wherein the detected characteristics include sleep characteristics comprising a blood pressure and a heart rate, and wherein the processor (305) is further configured to determine, based on the sleep characteristics, whether the patient is asleep.

6. The intra-oral device (100) of any one of claims 1 to 5, wherein the detected characteristics include apnea characteristics comprising a blood oxygen level, a heart rate, a mouth position, and a breathing activity, and wherein processor (305) is further configured to determine, based on the apnea characteristics, whether a sleep apnea occurs on the patient.

7. The intra-oral device (100) of any one of claims 1 to 6, wherein the detected characteristics include patient characteristics comprising a mandibular and head position, a position of the intra-oral device, and a breathing activity, wherein the processor (305) is configures to determine, based on the patient characteristics, when the patient is asleep.

8. A method of determining efficacy of using an intra-oral device (100) for reducing sleep apnea on a patient, wherein the intra-oral device comprises a lower arch component (120) and an upper arch component (110), wherein the lower arch component (120) is configured to be advanced relative to the upper arch component (110), the method comprising:
detecting, using sensors (150) disposed in the intra-oral device, characteristics of the patient,
wherein the characteristics comprise indicators of sleep apnea on the patient and the advancement of the lower arch component relative to the upper arch component,
**characterised in that**:
the sensors comprise a magnetometer (610) configured to detect a magnetic field generated by a magnet (620) disposed in the opposite arch component, and
the method comprises determining the advancement of the lower arch component relative to the upper arch component based on the detected magnetic field; and
determining efficacy of the intra-oral device in reducing the sleep apnea on the patient based on the detected characteristics and the determined advancement.

9. The method of claim 8, wherein the sensors (150) comprise a pulse oximeter sensor, an accelerometer, a temperature sensor, and a microphone.

10. The method of claim 8 or 9, further comprising determining compliance of the patient to using the intra-oral device according to a prescribed oral appliance therapy based on the detected characteristics.

11. The method of any one of claims 8 to 10, wherein the detected characteristics include use characteristics comprising a temperature, a blood pressure, a heart rate, and a blood oxygen level of the patient, and wherein the method further comprises determining, based on the use characteristics, whether the intra-oral device (100) is being used by the patient.

12. The method of any one of claims 8 to 11, wherein the detected characteristics include sleep characteristics comprising a blood pressure and a heart rate, wherein the method further comprises determining, based on the sleep characteristics, whether the patient is asleep.

13. The method of any one of claims 8 to 12, wherein the detected characteristics include apnea characteristics comprising a blood oxygen level, a heart rate, a mouth position, and a breathing activity, wherein the method further comprises determining, based on the apnea characteristics, whether a sleep apnea occurs on the patient.

14. The method of any one of claims 8 to 13, wherein the detected characteristics include patient characteristics comprising a mandibular and head position, a position of the intra-oral device, and a breathing activity, wherein method comprises determining, based on the patient characteristics, whether the patient is asleep.

15. A computer program product comprising a computer program, wherein when the computer program is executed by a processor (305), the processor is configured to perform the method according to any one of claims 8 to 14.

## Patentansprüche

1. Intraorale Vorrichtung (100), umfassend:
eine untere Bogenkomponente (120) und eine obere Bogenkomponente (110), wobei die untere Bogenkomponente (120) dazu konfiguriert ist, relativ zu der oberen Bogenkomponente (120) vorgeschoben zu werden;
Sensoren (150), die zum Detektieren von Eigenschaften eines Patienten konfiguriert sind,
wobei die Sensoren (150) in der oberen Bogenkomponente (110) oder der unteren Bogenkomponente (120) angeordnet sind, wobei die Eigenschaften Indikatoren für Schlafapnoe bei dem Patienten und den Vorschub der unteren Bogenkomponente (120) relativ zu der oberen Bogenkomponente (110) umfassen; und
einen Prozessor (305) in Kommunikation mit den Sensoren (150), um die detektierten Eigenschaften zu empfangen,
**dadurch gekennzeichnet, dass**:
die Sensoren (150) ein Magnetometer (610) umfassen, das dazu konfiguriert ist, ein Magnetfeld zu detektieren, das durch einen Magneten (620) erzeugt wird, der in der gegenüberliegenden Bogenkomponente angeordnet ist; und
wobei ferner der Prozessor (305) zu Folgendem konfiguriert ist:
Bestimmen, basierend auf dem detektierten Magnetfeld, des Vorschubs der unteren Bogenkomponente (120) relativ zu der oberen Bogenkomponente (110); und
Bestimmen, basierend auf den detektierten Eigenschaften und dem bestimmten Vorschub, der Wirksamkeit der intraoralen Vorrichtung (100) beim Reduzieren der Schlafapnoe des Patienten.

2. Intraorale Vorrichtung (100) nach Anspruch 1, wobei die Sensoren (150) einen Pulsoxymetersensor, einen Beschleunigungsmesser, einen Temperatursensor und ein Mikrofon umfassen.

3. Intraorale Vorrichtung (100) nach Anspruch 1 oder 2, wobei der Prozessor (305) ferner dazu konfiguriert ist, basierend auf den detektierten Eigenschaften Compliance des Patienten beim Verwenden der intraoralen Vorrichtung (100) gemäß einer vorgeschriebenen oralen Gerätetherapie zu bestimmen.

4. Intraorale Vorrichtung (100) nach einem der Ansprüche 1 bis 3, wobei die detektierten Eigenschaften Verwendungseigenschaften beinhalten, die eine Temperatur, einen Blutdruck, eine Herzfrequenz und einen Blutsauerstoffspiegel des Patienten umfassen, und wobei der Prozessor (305) ferner dazu konfiguriert ist, basierend auf den Verwendungseigenschaften zu bestimmen, ob die intraorale Vorrichtung (100) durch den Patienten verwendet wird.

5. Intraorale Vorrichtung (100) nach einem der Ansprüche 1 bis 4, wobei die detektierten Eigenschaften Schlafeigenschaften beinhalten, die einen Blutdruck und eine Herzfrequenz umfassen, und wobei der Prozessor (305) ferner dazu konfiguriert ist, basierend auf den Schlafeigenschaften zu bestimmen, ob der Patient schläft.

6. Intraorale Vorrichtung (100) nach einem der Ansprüche 1 bis 5, wobei die detektierten Eigenschaften Apnoeeigenschaften beinhalten, die einen Blutsauerstoffspiegel, eine Herzfrequenz, eine Mundposition und eine Atemaktivität umfassen, und wobei der Prozessor (305) ferner dazu konfiguriert ist, basierend auf den Apnoeeigenschaften zu bestimmen, ob eine Schlafapnoe bei dem Patienten auftritt.

7. Intraorale Vorrichtung (100) nach einem der Ansprüche 1 bis 6, wobei die detektierten Eigenschaften Patienteneigenschaften beinhalten, die eine Unterkiefer- und Kopfposition, eine Position der intraoralen Vorrichtung und eine Atemaktivität umfassen, wobei der Prozessor (305) dazu konfiguriert ist, basierend auf den Patienteneigenschaften zu bestimmen, wann der Patient schläft.

8. Verfahren zum Bestimmen von Wirksamkeit des Verwendens einer intraoralen Vorrichtung (100) zum Reduzieren von Schlafapnoe bei einem Patienten, wobei die intraorale Vorrichtung eine untere Bogenkomponente (120) und eine obere Bogenkomponente (110) umfasst, wobei die untere Bogenkomponente (120) dazu konfiguriert ist, relativ zu der oberen Bogenkomponente (110) vorgeschoben zu werden, wobei das Verfahren Folgendes umfasst:
Detektieren, unter Verwendung von Sensoren (150), die in der intraoralen Vorrichtung angeordnet sind, von Eigenschaften des Patienten,
wobei die Eigenschaften Indikatoren für Schlafapnoe bei dem Patienten und den Vorschub der unteren Bogenkomponente relativ zu der oberen Bogenkomponente umfassen,
**dadurch gekennzeichnet, dass**:
die Sensoren ein Magnetometer (610) umfassen, das dazu konfiguriert ist, ein Magnetfeld zu detektieren, das durch einen Magneten (620) erzeugt wird, der in der gegenüberliegenden Bogenkomponente angeordnet ist, und
das Verfahren Bestimmen des Vorschubs der unteren Bogenkomponente relativ zu der oberen Bogenkomponente basierend auf dem detektierten Magnetfeld umfasst; und
Bestimmen von Wirksamkeit der intraoralen Vorrichtung beim Reduzieren der Schlafapnoe bei dem Patienten basierend auf den detektierten Eigenschaften und dem bestimmten Vorschub.

9. Verfahren nach Anspruch 8, wobei die Sensoren (150) einen Pulsoxymetersensor, einen Beschleunigungsmesser, einen Temperatursensor und ein Mikrofon umfassen.

10. Verfahren nach Anspruch 8 oder 9, ferner umfassend Bestimmen von Compliance des Patienten beim Verwenden der intraoralen Vorrichtung gemäß einer verschriebenen oralen Gerätetherapie basierend auf den detektierten Eigenschaften.

11. Verfahren nach einem der Ansprüche 8 bis 10, wobei die detektierten Eigenschaften Verwendungseigenschaften beinhalten, die eine Temperatur, einen Blutdruck, eine Herzfrequenz und einen Blutsauerstoffgehalt des Patienten umfassen, und wobei das Verfahren ferner Bestimmen, basierend auf den Verwendungseigenschaften, ob die intraorale Vorrichtung (100) durch den Patienten verwendet wird, umfasst.

12. Verfahren nach einem der Ansprüche 8 bis 11, wobei die detektierten Eigenschaften Schlafeigenschaften beinhalten, die einen Blutdruck und eine Herzfrequenz umfassen, wobei das Verfahren ferner Bestimmen, basierend auf den Schlafeigenschaften, ob der Patient schläft, umfasst.

13. Verfahren nach einem der Ansprüche 8 bis 12, wobei die detektierten Eigenschaften Apnoeeigenschaften beinhalten, die einen Blutsauerstoffspiegel, eine Herzfrequenz, eine Mundposition und eine Atemaktivität umfassen, wobei das Verfahren ferner Bestimmen, basierend auf den Apnoeeigenschaften, ob eine Schlafapnoe bei dem Patienten auftritt, umfasst.

14. Verfahren nach einem der Ansprüche 8 bis 13, wobei die detektierten Eigenschaften Patienteneigenschaften beinhalten, die eine Unterkiefer- und Kopfposition, eine Position der intraoralen Vorrichtung und eine Atemaktivität umfassen, wobei das Verfahren Bestimmen, basierend auf den Patienteneigenschaften, ob der Patient schläft, umfasst.

15. Computerprogrammprodukt, umfassend ein Computerprogramm, wobei, wenn das Computerprogramm durch einen Prozessor (305) ausgeführt wird, der Prozessor dazu konfiguriert ist, das Verfahren nach einem der Ansprüche 8 bis 14 durchzuführen.

## Revendications

1. Dispositif intra-oral (100) comprenant :
un composant de voûte inférieure (120) et un composant de voûte supérieure (110), dans lequel le composant de voûte inférieure (120) est conçu pour être avancé par rapport au composant de voûte supérieure (120) ;
des capteurs (150) conçus pour détecter des caractéristiques d'un patient, dans lequel les capteurs (150) sont disposés dans le composant de voûte supérieure (110) ou le composant de voûte inférieure (120), dans lequel les caractéristiques comprennent des indicateurs d'apnée du sommeil chez le patient et l'avancée du composant de voûte inférieure (120) par rapport au composant de voûte supérieure (110) ; et
un processeur (305) en communication avec les capteurs (150) pour recevoir les caractéristiques détectées,
**caractérisé en ce que** :
les capteurs (150) comprennent un magnétomètre (610) conçu pour détecter un champ magnétique généré par un aimant (620) disposé dans le composant de voûte opposée ; et
dans lequel le processeur (305) est en outre conçu pour :
déterminer, sur la base du champ magnétique détecté, l'avancée du composant de voûte inférieure (120) par rapport au composant de voûte supérieure (110) ; et
déterminer, sur la base des caractéristiques détectées et de l'avancée déterminée, l'efficacité du dispositif intra-oral (100) dans la réduction de l'apnée du sommeil du patient.

2. Dispositif intra-oral (100) de la revendication 1, dans lequel les capteurs (150) comprennent un capteur oxymètre de pouls, un accéléromètre, un capteur de température et un microphone.

3. Dispositif intra-oral (100) de la revendication 1 ou 2, dans lequel le processeur (305) est en outre conçu pour déterminer, sur la base des caractéristiques détectées, l'adéquation du patient à l'utilisation du dispositif intra-oral (100) selon une thérapie par appareil oral prescrite.

4. Dispositif intra-oral (100) de l'une quelconque des revendications 1 à 3, dans lequel les caractéristiques détectées comprennent des caractéristiques d'utilisation comprenant une température, une pression artérielle, une fréquence cardiaque et un niveau d'oxygène sanguin du patient, et dans lequel le processeur (305) est en outre conçu pour déterminer, sur la base des caractéristiques d'utilisation, si le dispositif intra-oral (100) est utilisé par le patient.

5. Dispositif intra-oral (100) de l'une quelconque des revendications 1 à 4, dans lequel les caractéristiques détectées comprennent des caractéristiques de sommeil comprenant une pression artérielle et une fréquence cardiaque, et dans lequel le processeur (305) est en outre conçu pour déterminer, sur la base des caractéristiques de sommeil, si le patient est endormi.

6. Dispositif intra-oral (100) de l'une quelconque des revendications 1 à 5, dans lequel les caractéristiques détectées comprennent des caractéristiques d'apnée comprenant un niveau d'oxygène sanguin, une fréquence cardiaque, une position de la bouche et une activité respiratoire, et dans lequel le processeur (305) est en outre conçu pour déterminer, sur la base des caractéristiques d'apnée, si une apnée du sommeil se produit chez le patient.

7. Dispositif intra-oral (100) de l'une quelconque des revendications 1 à 6, dans lequel les caractéristiques détectées comprennent des caractéristiques de patient comprenant une position de la mandibule et de la tête, une position du dispositif intra-oral et une activité respiratoire, dans lequel le processeur (305) est conçu pour déterminer, sur la base des caractéristiques de patient, quand le patient est endormi.

8. Procédé de détermination de l'efficacité de l'utilisation d'un dispositif intra-oral (100) pour réduire l'apnée du sommeil chez un patient, dans lequel le dispositif intra-oral comprend un composant de voûte inférieure (120) et un composant de voûte supérieure (110), dans lequel le composant de voûte inférieure (120) est conçu pour être avancé par rapport au composant de voûte supérieure (110), le procédé comprenant :
la détection, à l'aide de capteurs (150) disposés dans le dispositif intra-oral, de caractéristiques du patient,
dans lequel les caractéristiques comprennent des indicateurs d'apnée du sommeil chez le patient et l'avancée du composant de voûte inférieure par rapport au composant de voûte supérieure, **caractérisé en ce que** :
les capteurs comprennent un magnétomètre (610) conçu pour détecter un champ magnétique généré par un aimant (620) disposé dans le composant de voûte opposée, et
le procédé comprend la détermination de l'avancée du composant de voûte inférieure par rapport au composant de voûte supérieure sur la base du champ magnétique détecté ; et
la détermination de l'efficacité du dispositif intra-oral dans la réduction de l'apnée du sommeil chez le patient sur la base des caractéristiques détectées et de l'avancée déterminée.

9. Procédé de la revendication 8, dans lequel les capteurs (150) comprennent un capteur oxymètre de pouls, un accéléromètre, un capteur de température et un microphone.

10. Procédé de la revendication 8 ou 9, comprenant en outre la détermination de l'adéquation du patient à l'utilisation du dispositif intra-oral selon une thérapie par appareil oral prescrite sur la base des caractéristiques détectées.

11. Procédé de l'une quelconque des revendications 8 à 10, dans lequel les caractéristiques détectées comprennent des caractéristiques d'utilisation comprenant une température, une pression artérielle, une fréquence cardiaque et un niveau d'oxygène sanguin du patient, et dans lequel le procédé comprend en outre le fait de déterminer, sur la base des caractéristiques d'utilisation, si le dispositif intra-oral (100) est utilisé par le patient.

12. Procédé de l'une quelconque des revendications 8 à 11, dans lequel les caractéristiques détectées comprennent des caractéristiques de sommeil comprenant une pression artérielle et une fréquence cardiaque, dans lequel le procédé comprend en outre le fait de déterminer, sur la base des caractéristiques de sommeil, si le patient est endormi.

13. Procédé de l'une quelconque des revendications 8 à 12, dans lequel les caractéristiques détectées comprennent des caractéristiques d'apnée comprenant un niveau d'oxygène sanguin, une fréquence cardiaque, une position de la bouche et une activité respiratoire, dans lequel le procédé comprend en outre le fait de déterminer, sur la base des caractéristiques d'apnée, si une apnée du sommeil se produit chez le patient.

14. Procédé de l'une quelconque des revendications 8 à 13, dans lequel les caractéristiques détectées comprennent des caractéristiques de patient comprenant une position de la mandibule et de la tête, une position du dispositif intra-oral et une activité respiratoire, dans lequel le procédé comprend le fait de déterminer, sur la base des caractéristiques de patient, si le patient est endormi.

15. Produit de programme informatique comprenant un programme informatique, dans lequel, lorsque le programme informatique est exécuté par un processeur (305), le processeur est configuré pour réaliser le procédé selon l'une quelconque des revendications 8 à 14.
